# EUROPEAN PATENT SPECIFICATION

(11) **EP 3 415 159 B1**
(45) Date of publication and mention of the grant of the patent: **13.12.2023**
(21) Application number: 17750278.8
(22) Date of filing: 08.02.2017
(51) Int. Cl.: A61K 39/00, A61P 43/00, A61P 37/04, A61P 35/00, A61K 47/36, A61K 45/00, A61K 35/17, A61K 9/14, A01K 67/027, A61K 9/00, A61K 9/51, A61K 45/06

(54) **PRETREATMENT DRUG FOR T CELL INFUSION THERAPY FOR IMMUNE-CHECKPOINT INHIBITOR-RESISTANT TUMOR**
VORBEHANDLUNGSARZNEIMITTEL FÜR T-ZELLINFUSIONSTHERAPIE FÜR IMMUNCHECKPOINTINHIBITORRESISENTEN TUMOR
MÉDICAMENT DE PRÉTRAITEMENT POUR THÉRAPIE PAR PERFUSION DE LYMPHOCYTES T POUR TUMEUR RÉSISTANTE AUX INHIBITEURS DE POINT DE CONTRÔLE IMMUNITAIRE

(30) Priority: 08.02.2016 JP 2016022081
(43) Date of publication of application: 19.12.2018
(73) Proprietor: Mie University, Tsu-shi, Mie 514-8507 (JP); Kyoto University, Kyoto-shi, Kyoto 606-8501 (JP)
(72) Inventor: SHIKU, Hiroshi, Tsu-shi Mie 514-8507 (JP); HARADA, Naozumi, Tsu-shi Mie 514-8507 (JP); MURAOKA, Daisuke, Tsu-shi Mie 514-8507 (JP); AKIYOSHI, Kazunari, Kyoto-shi Kyoto 606-8501 (JP)
(74) Representative: Mewburn Ellis LLP
(86) International application number: PCT/JP2017/004552
(87) International publication number: WO 2017/138557

(56) References cited:
- WO-A1-2013/031882
- WO-A1-2015/050158
- WO-A1-2015/050158
- SHIMIZU TAKESHI ET AL: "Nanogel DDS enables sustained release of IL-12 for tumor immunotherapy", BIOCHEMICAL AND BIOPHYSICAL RESEARCH COMMUNICATIONS, vol. 367, no. 2, 26 December 2007 (2007-12-26), pages 330-335, XP029231169, ISSN: 0006-291X, DOI: 10.1016/J.BBRC.2007.12.112
- SHIMODA ASAKO ET AL: "Dual crosslinked hydrogel nanoparticles by nanogel bottom-up method for sustained-release delivery", COLLOIDS AND SURFACES. B, BIOINTERFACES, vol. 99, 19 September 2011 (2011-09-19), pages 38-44, XP028928711, ISSN: 0927-7765, DOI: 10.1016/J.COLSURFB.2011.09.025
- J. MITSUI ET AL: "Two Distinct Mechanisms of Augmented Antitumor Activity by Modulation of Immunostimulatory/Inhibitory Signals", CLINICAL CANCER RESEARCH, vol. 16, no. 10, 15 May 2010 (2010-05-15), pages 2781-2791, XP055100557, ISSN: 1078-0432, DOI: 10.1158/1078-0432.CCR-09-3243
- DAISUKE MURAOKA ET AL: "Nanogel-Based Immunologically Stealth Vaccine Targets Macrophages in the Medulla of Lymph Node and Induces Potent Antitumor Immunity", ACS NANO, vol. 8, no. 9, 23 September 2014 (2014-09-23), pages 9209-9218, XP055357955, US ISSN: 1936-0851, DOI: 10.1021/nn502975r
- CARL H. JUNE: "Adoptive T cell therapy for cancer in the clinic", JOURNAL OF CLINICAL INVESTIGATION, vol. 117, no. 6, 1 June 2007 (2007-06-01), pages 1466-1476, XP055287551, GB ISSN: 0021-9738, DOI: 10.1172/JCI32446
- NAOKO IMAI et al.: "Dai 66 Kai Gan Men'eki Ryoho no Bunshi Kiko 3 CD 8+ Saibo Shogaisei T Saibo (CTL) no Seigyo ni yoru Gan Men'eki Ryoho -T Saibo Yuchu Ryoho o Chushin ni", Surgery Frontier, vol. 17, no. 3, 2010, pages (263) 65-(268) 70, XP009515225, ISSN: 1340-5594
- NAOKO IMAI et al.: "Tokushu: Kokei Gan no Men'eki·Kotai Ryoho, III. Rinsho Oyo no Shinpo to Tenbo Men'eki Ryoho (Men'eki Saibo Ryoho), Targeting cancer antigen (MAGE-A4, NY -ESO-1) for immunotherapy", Japanese Journal of Clinical Medicine, vol. 70, no. 12, 2012, pages 2125-2129, XP009513074, ISSN: 0047-1852
- YOZO NAKAZAWA et al.: "Gene-modified T-cell Therapy Using Chimeric Antigen Receptor", The Shinshu Medical Journal, vol. 61, no. 4, 2013, pages 197-203, XP055516593,
- HIROSHI SHIKU et al.: "Kicho Koen 1 Gan Men'eki Ryoho -Gan Vaccine, T Saibo Yuchu Ryoho, Soshite Fukugoteki Men'eki Ryoho", Dai 66 Kai The Japanese Urological Association Chubu Sokai, 27 October 2016 (2016-10-27), page 83, XP9515187,
- KAGEYAMA, S. et al.: "Dose-dependent effects of NY -ESO-1 protein vaccine complexed with cholesteryl pullulan (CHP- NY -ESO-1) on immune responses and survival benefits of esophageal cancer patients", J. Translational Medicine, vol. 11, no. 246, 2013, pages 1-10, XP021164407,

## Description

### [Technical Field]

The invention relates to a pretreatment drug which enhances the efficacy of T cell infusion therapy against immune checkpoint inhibitor-resistant tumors.

### [Background Art]

T cells play important roles in tumor immune response. T cells recognize antigen protein-derived epitope peptides bound to major histocompatibility complex (MHC) presented on the surface of antigen presenting cells (dendritic cells, macrophages, etc.) with T cell receptor (TCR) expressed on the surface of T cells. The reaction is called antigen stimulation. Simultaneously with antigen stimulation, co-stimulatory signal is generated by binding between a membrane protein CD28 on T cells and a membrane protein CD80 or CD86 on antigen presenting cells. T cells are suitably activated by TCR signal via antigen stimulation and co-stimulatory signal.

In contrast, there is a regulatory mechanism so called immune checkpoint to prevent excessive reaction of T cells. A membrane protein CTLA-4 is expressed on activated T cells and binds to CD80 or CD86 on antigen presenting cells. As a result, the binding inhibits the binding of CD28 and CD80, or CD28 and CD86, and prevents the establishment of co-stimulatory signal, and inputs an inhibitory signal into T cell. CTLA-4 expressed on regulatory T cells binds to CD80 or CD86 on antigen presenting cells, and thereby suppresses the activity of antigen presenting cells. Through these effects, CTLA-4 acts an immune checkpoint molecule to suppress the action of T cells.

A membrane protein PD-1 upregulated by activation of T cells is a kind of immune checkpoint molecule. PD-L1 is known as a ligand which binds to PD-1. PD-L1 is expressed on many tumor cells and activated immune cells. When PD-L1 binds to PD-1 on T cells, TCR signal upon antigen stimulation is inhibited by PD-1 signal. As a result, cytokine production from and cytotoxicity of T cells are reduced. PD-1 signal may suppress the proliferation and survival of T cells.

Immune checkpoint molecules such as CTLA-4, PD-1 and PD-L1 weaken the action of tumor-specific T cells. As a result, these molecules are one of main causes for tumor to escape from immunity. By inhibiting the action of CTLA-4, PD-1 or PD-L1, the function of tumor-specific T cells can be recovered, and the immune attack to tumor can be enhanced. The use of inhibitors of immune checkpoint molecules has been evaluated in a variety of human cancers. CTLA-4 inhibitory antibody and PD-1 inhibitory antibody show superior therapeutic effects, such as tumor regression and prolonged survival, in refractory melanoma, lung cancer and renal cell carcinoma patients. However, the response rate is only 20 to 30 percent in any of the cancer types. Many cancer patients are resistant to immune checkpoint inhibitors. Development of effective treatments in cancer patients who are resistant to immune checkpoint inhibitors has become an important issue in cancer treatment.

Some Candidates for the effective treatment of immune checkpoint inhibitor-resistant tumors were found using an in vitro testing system. A combination therapy of intratumoral administration of oncolytic virus (Newcastle disease virus) and anti-CTLA-4 antibody shows a therapeutic effect on nonclinical tumor model in which a mouse melanoma cell line B16F10, mouse prostate cancer cell line TRAMP-C2, or mouse colon cancer cell line CT26 is implanted subcutaneously into wild type mice. In this condition, anti-CTLA-4 antibody alone does not have any therapeutic effect (Zamarin, D., et al. Sci. Transl. Med. 2014;6(226):226-32). A combination therapy of tumor cell vaccine transduced with a GM-CSF gene and treated with radiation and STING agonist and anti-PD-1 antibody shows a therapeutic effect on nonclinical tumor model in which a mouse melanoma cell line B16F10 or mouse colon cancer cell line CT26 is implanted subcutaneously into wild type mice. In this condition, anti-PD-1 antibody does not have any therapeutic effect (Fu, J., et al. Sci. Transl. Med. 2015;7(283):283-52). A combination therapy of 4 drugs including DNA methylation inhibitor, HDAC inhibitor, anti-CTLA-4 antibody, and anti-PD-1 antibody shows a therapeutic effect on nonclinical tumor model in which a mouse breast cancer cell line 4T1 is implanted subcutaneously into wild type mice. In this condition, a combination therapy of anti-CTLA-4 antibody and anti-PD-1 antibody does not have any therapeutic effect (Kim, K., et al. Proc. Natl. Acad. Sci. U.S.A. 2014;111(32):11774-9). A combination therapy of human Her2-specific chimeric antigen receptor (CAR)-engineered T cell infusion and anti-PD-1 antibody shows a therapeutic effect on nonclinical tumor model in which a murine sarcoma cell line 24JK expressing human Her2 antigen was implanted subcutaneously into a human Her2 transgenic mice. In this condition, anti-PD-1 antibody alone does not have any therapeutic effect (John, L. B., et al. Clin. Cancer Res. 2013;19(20):5636-46). These reports are characterized by combining immune checkpoint inhibitors and other anti-cancer agents. Therapeutic effects on tumor are observed only in animal tumor models that express molecular targets of immune checkpoint inhibitors.

In human cancers, the mechanisms of resistance to immune checkpoint inhibitors have been elucidated. An analysis of tumor tissues of melanoma patients, who exhibit sensitivity or resistance to anti-PD-1 antibody, showed that the expression of PD-L1 and PD-1 in the tumor was significantly lower in the patients with resistance (Tumeh, P. C., et al. Nature. 2014;515 (7528):568-71). The results indicate that the lack of expression of molecular targets of immune checkpoint inhibitors at the tumor site is a cause of the resistance to the inhibitors. Treatment methods shown in the aforementioned examples are characterized by combining immune checkpoint inhibitors and other anti-cancer agents. These treatments are effective against tumors that express molecular targets of immune checkpoint inhibitors. However, these therapies may be less effective against tumors that do not express the molecular targets of immune checkpoint inhibitors. These results show that novel therapies are needed for tumors that do not express the molecular targets of immune checkpoint inhibitors. Shimizu Takeshi et al., 2008, (Biochemical and Bioph. Res. Comm. vol. 367, no. 2 , pages 330-335), discloses a delivery system using cholesterol bearing pullanan (CHP)-based hydrogel nanoparticals (nanogel) which comprises IL-12 protein. Such particles have a size of 20-30nm after self-aggregation and was used intravenously. Simoda Asoka et al., 2012, (Colloids and Surfaces. B, Biointerfaces, vol. 99 , pages 38-44), discloses the inoculation of mice with CMS5a cells as a model to investigate certain aspects of antitumor activity by specified antibodies against immune checkpoint inhibitors. Daisuke Maraoka et al., 2014, (ACS NANO, vol. 8, no. 9, pages 9209-9218) and WO2015/050158 discloses the use of CHP nanogel in subcutaneous injections. The nanogel contains a long-peptide antigen. After injection the nanoparticles arrive at macrophages in the lymph-nodes close to the tumors, which is also here based on CMS5a and therefore a immune checkpoint inhibitor-resistant tumor. A potent antitumor immunity is induced by this procedure. Carl June, 2007, JOURNAL OF CLINICAL INVESTIGATION, vol. 117, no. 6, pages 1466-1476, discloses various systems for the use of patient derived T-cells in the treatment of cancer.

### [SUMMARY OF THE INVENTION]

The disclosure relates to a therapeutic technique for treating immune checkpoint inhibitor-resistant tumors. Specifically, a very effective pretreatment anti-tumor drug (antigen-loaded nanogel and immunological enhancer) combined with T cell infusion therapy is provided.

The present inventors have studied an effective therapy for immune checkpoint inhibitors-resistant tumors in which the expression of molecular target of immune checkpoint inhibitors is low at the tumor site. As a pretreatment drug, a hydrophobized polysaccharide-based nanogel in which a synthetic long peptide antigen or a recombinant protein antigen is loaded and immune-enhancing agent were used. The disclosure is based on the finding that the infusion of antigen-specific T cells had a remarkable effect on tumors that are resistant to immune checkpoint inhibitors.

The invention herein is as defined by the claims.

In the disclosure, the long peptide antigen or the protein antigen can be used the recombinant protein antigen. In this case, nucleic acids having the nucleotide sequence encoding the recombinant protein which contains a given amino acid sequence are prepared, after the recombinant proteins are expressed using the cells (eukaryotic or prokaryotic) incorporated the nucleic acids, the recombinant protein antigen can be purified by known methods.

With the disclosure, a useful pharmaceutical composition for treating tumors that does not express molecular targets immune checkpoint inhibitors and are resistant to the immune checkpoint inhibitors is provided. The enhancement of anti-cancer effect of antigen-specific T cell infusion is derived by using an antigen-loaded nanogel which contains a hydrophobized polysaccharide-based nanogel as a delivery system and a synthetic long peptide antigen or a recombinant protein antigen and immune-enhancing agent, as a pretreatment drug.

### [BRIEF DESCRIPTION OF THE FUGRES]

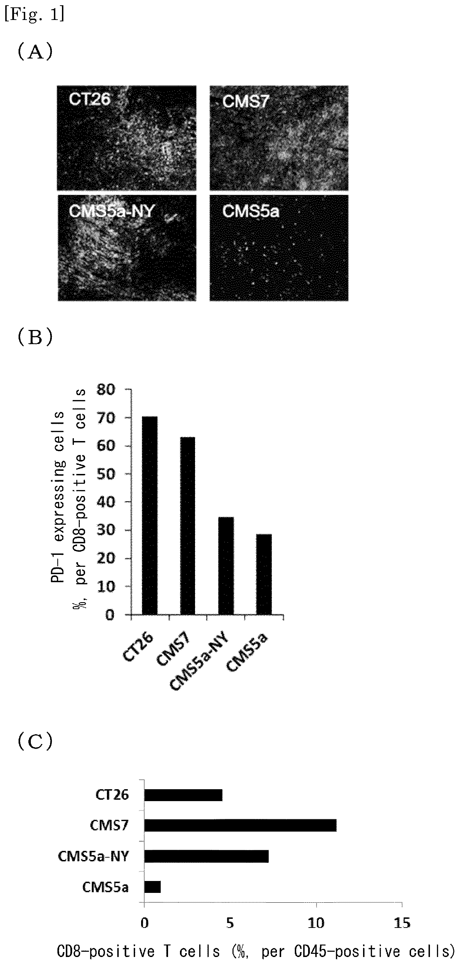

Fig. 1 illustrates the data indicating the expression of PD-L1 and PD-1, and numbers of CD8-positive T cells infiltrating into tumor for various mouse tumors implanted subcutaneously and engrafted into BALB/c mice. (A) shows a photomicrograph showing the results of analyzing the expression of PD-L1 molecules in tumor locally after 7 days from implanted, (B) shows a graph showing the results of analyzing the PD-1 expression of CD3-positive T cells localized in each tumor by flow cytometry, (C) shows a graph showing the results of the analyzing the number of CD8-positive T cells infiltrated into each tumor.

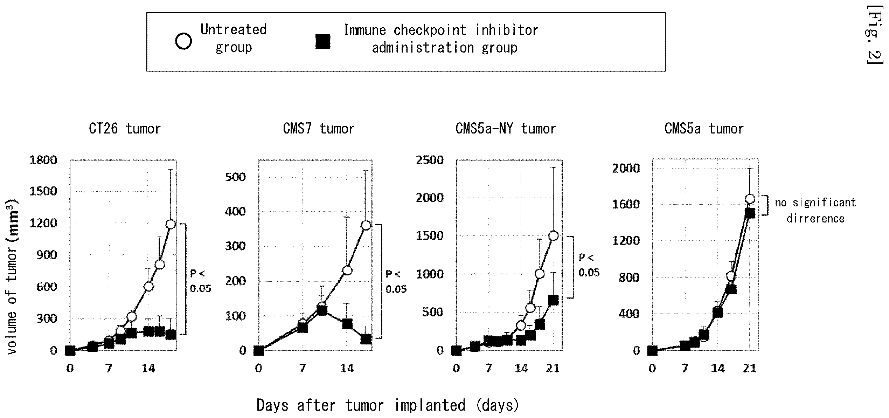

Fig. 2 illustrates graphs showing the results of examining the susceptibility to immune checkpoint inhibitors of various mouse tumors implanted subcutaneously and engrafted into BALB/c mice.

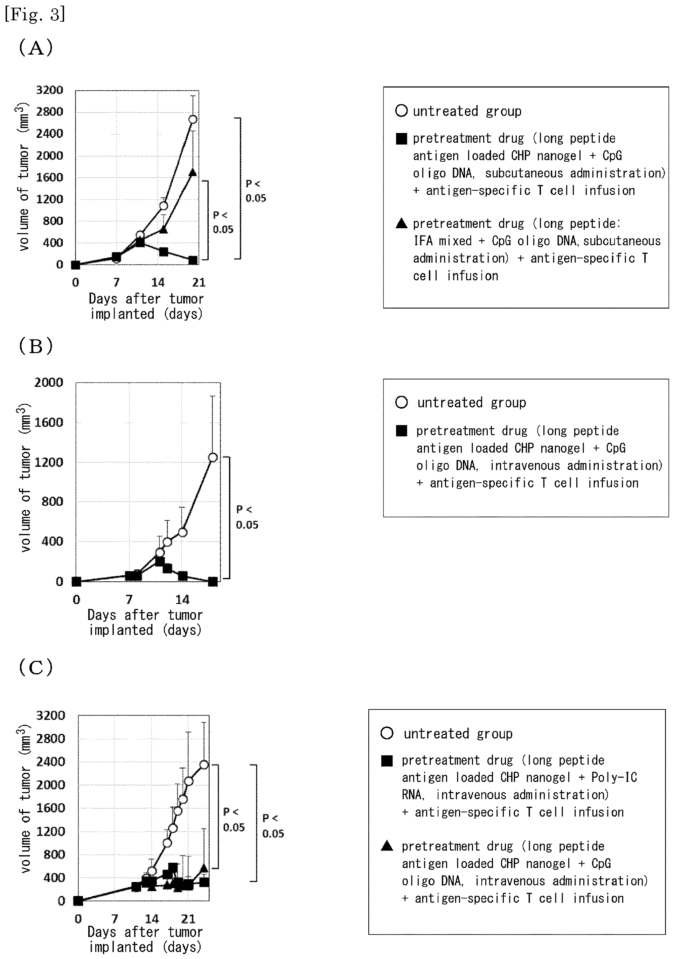

Fig. 3 illustrates graphs showing the results of the therapeutic efficacy of antigen-specific T cell infusion to the BALB/c mice which was subcutaneously transplanted with fibrosarcoma CMS5a tumors, using the pretreatment drug which contains the long peptide antigen-loaded cholesteryl pullulan (CHP) nanogel and immune-enhancing agent. (A) shows a graph showing that the antigen-specific T cell infusion after subcutaneous administration of the long peptide antigen-loaded CHP nanogel and CpG oligo DNA can heal CMS5a tumor, and that incomplete Freund's adjuvant (IFA) instead of the nanogel as the delivery system can not heal, (B) shows a graph showing that the antigen-specific T cell infusion after intravenous administration of the long peptide antigen-loaded CHP nanogel and CpG oligo DNA can heal CMS5a tumor, and that intravenous administration of the antigen-loaded nanogel of the disclosure has the same effect as subcutaneous administration, (C) shows a graph showing that the antigen-specific T cell infusion after the administration of the long peptide antigen-loaded CHP nanogel and poly-IC RNA can heal CMS5a tumor, and that poly-IC RNA used as immune-enhancing agent has the same effect as CpG oligo DNA.

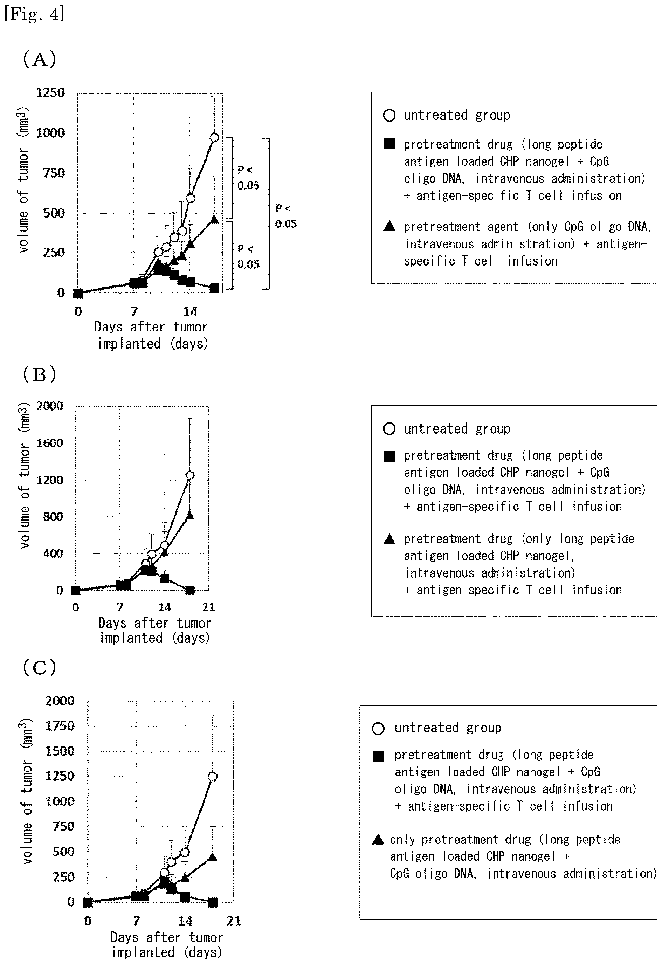

Fig. 4 illustrates graphs showing the results of the therapeutic efficacy of antigen-specific T cell infusion to the BALB/c mice which was subcutaneously transplanted with CMS5a tumors, using the pretreatment drug which contains the long peptide antigen loaded CHP nanogel and immune-enhancing agent. (A) shows a graph showing that the antigen-specific T cell infusion after the administration of the long peptide antigen loaded CHP nanogel and CpG oligo DNA can heal CMS5a tumor, and that CpG oligo DNA omitting the long peptide antigen-loaded CHP nanogel cannot heal, (B) shows a graph showing that the antigen-specific T cell infusion after the administration of the long peptide antigen-loaded CHP nanogel and CpG oligo DNA can heal CMS5a tumor, and that the long peptide antigen-loaded CHP nanogel without CpG oligo DNA can not heal, (C) shows a graph showing that the antigen-specific T cell infusion after the administration of the long peptide antigen-loaded CHP nanogel and CpG oligo DNA can heal CMS5a tumor, and that the long peptide antigen-loaded CHP nanogel and CpG oligo DNA without the antigen-specific T cell infusion can not heal.

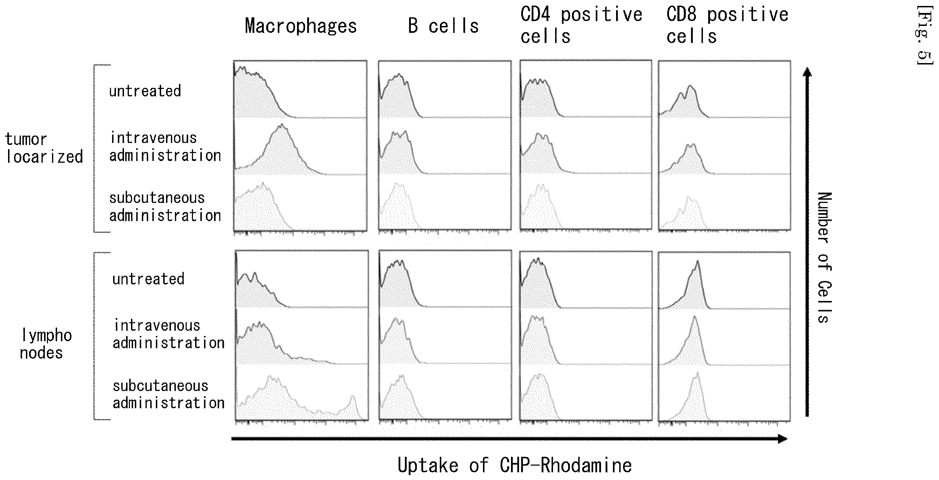

Fig. 5 illustrates the data showing the results of incorporation assay of CHP nanogel to tumor localized immune cells when the CHP nanogel was administered intravenously to BALB/c mice in which CMS5a tumor was implanted subcutaneously.

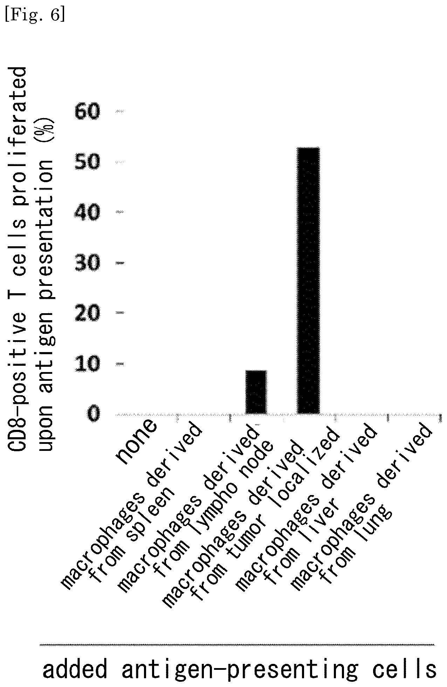

Fig. 6 illustrates the data showing the results of analysis on antigen presenting activity of tumor localized macrophages when the long chain antigen-loaded CHP nanogels and CpG oligo DNA were administered to BALB/c mice in which CMS5a tumor was implanted subcutaneously.

### [DESCRIPTION OF THE PREFERRED EMBODIMENTS]

### Embodiments of the pretreatment drug

The pretreatment drug of the disclosure is characterized that it comprises one or more immune-enhancing agent and a pharmaceutical composition, which contains a hydrophobized polysaccharide-based nanogel as a delivery system in which a synthetic long peptide antigen or a recombinant protein antigen is loaded, wherein the long peptide antigen or the protein antigen contains simultaneously CD8-positive cytotoxic T cell recognizing epitope(s) and/or CD4-positive helper T cell recognizing epitope(s) derived from a tumor-specific antigen protein or a tumor stroma-specific antigen.

The synthetic long peptide antigen preferably contains 23 to 120 amino acid residues and at least two T cell recognizing epitopes. The synthetic long peptide antigen preferably contains 23 to 80 amino acids and at least two T cell recognizing epitopes. The synthetic long peptide antigen preferably contains 23 to 60 amino acids and at least two T cell recognizing epitopes.

The recombinant protein antigen preferably contains two or more T cell recognizing epitopes and a tag sequence for purification as necessary, and is a full-length or partial-length antigen protein produced in E. coli., insect cell or mammalian cell.

The CD8-positive cytotoxic T cell recognizing epitope is preferably a portion of the amino acid sequence of a tumor-specific antigen protein or a tumor stroma-specific antigen protein. The CD4-positive helper T cell recognizing epitope is preferably a portion of the amino acid sequence of a tumor-specific antigen protein or a tumor stroma-specific antigen protein.

The tumor-specific antigen protein is preferably selected from the group consisting of MAGE family, NY-ESO-1/LAGE, SAGE, XAGE, HER2, PRAME, Ras, 5T4, WT1, p53, MUC-1, hTERT, RHAMM, Survivin, EGFRvIII, HPV E6, MART-1, gp100, CEA, IDO, Brachyury, Mesothelin, PSA and PSMA. The tumor stroma-specific antigen protein is preferably selected from the group consisting of FAP, VEGFR family and TEM1.

The polysaccharide constituting the hydrophobized polysaccharide-based nanogels is preferably a pullulan or mannan. The hydrophobic group of the hydrophobized polysaccharide-based nanogels is preferably a cholesterol. The hydrophobized polysaccharide-based nanogels is preferably a non-ionic. The particle size of the hydrophobized polysaccharide-based nanogels is preferably at 80 nm or less.

The immune-enhancing agent preferably contains a soluble TLR agonist, a soluble STING agonist or a soluble RLR agonist. As a soluble TLR agonist, CpG oligo DNA or poly-IC RNA is exemplified. As a soluble STING agonist, cyclic dinucleotide such as CdGMP, xanthenone-derivative such as DMXAA is exemplified. As a soluble RLR agonist, 5'-phosphorylated double-stranded RNA is exemplified.

In the disclosure, the synthetic long peptide antigen or the recombinant protein antigen is characterized in that it comprises at least two or more T cell recognizing epitopes contained in a tumor-specific antigen protein and/or a tumor stroma-specific antigen protein. T cell recognizing epitope is preferably one in a tumor-specific antigen protein or a tumor stroma-specific antigen protein. As such, it may be selected from MAGE family molecules such as MAGE-A1, MAGE-A2, MAGE-A3, MAGE-A4, MAGE-A5, MAGE-A6, MAGE-A8, MAGE-A9, MAGE-A10, MAGE-A11, MAGE-A12, MAGE-B1 and MAGE-B2, or T cell recognizing epitope contained in a tumor-specific antigen protein such as NY-ESO-1/LAGE molecule, SAGE, XAGE, HER2, PRAME, Ras, 5T4, WT1, p53, MUC-1, hTERT, RHAMM, Survivin, EGFRvIII, HPV E6, MART-1, gp100, CEA, IDO, Brachyury, Mesothelin, PSA and PSMA, or T cell recognizing epitope contained in a tumor stroma-specific antigen protein such as FAP, VEGFR family and TEM1. In T cell recognizing epitopes, there are CTL epitopes recognized by CD8-positive cytotoxic T cells and Th epitopes recognized by CD4-positive helper T cells. The synthetic long peptide antigen or the recombinant protein antigen in the disclosure preferably contains simultaneously more than one of the CTL epitopes and Th epitopes, respectively. The long peptide antigen containing a CTL epitope and the long peptide antigen containing a Th epitope can be used alone or in combination.

Hydrophobized polysaccharide used in the disclosure can be prepared by known methods. About polysaccharides in hydrophobized polysaccharide, polymer in which sugar residues are glycosidically bound can be used without limitation. The sugar residues constituting the polysaccharide may be used, for example, derived from mono-saccharide such as glucose, mannose, galactose, and fucose, or di-saccharide or oligosaccharide. Sugar residues, may be 1,2-, 1,3-, 1,4- or 1,6-glycosidic bond, the bond may be any of α-type bond or a β-type bond. The polysaccharide may be linear or branched. As a sugar residue, glucose residue may be preferably used, pullulan, dextran, amylose, amylopectin, or mannan of natural or synthetic origin may be used as a polysaccharide, preferably mannan or pullulan can be used. Average molecular weight of the polysaccharide can range from 50,000 to 150,000.

As the hydrophobic group, for example, in single-stranded and double-stranded chain, alkyl or sterol residues which are introduced at a rate of 1 to 5 per 100 monosaccharides (less than 5% by weight) are preferably used, at a rate of 1 to 3 per 100 monosaccharides (less than 3% by weight) are more preferably used. As the hydrophobic group, an alkyl group or a sterol residue is not limited, other residues can be used with good efficiency depending on the molecular weight or the isoelectric point of an antigen encapsulated. As the sterol residue, such as cholesterol, stigmasterol, beta-sitosterol, lanosterol and ergosterol residues are exemplified. Preferably, a cholesterol residue is used. As the alkyl group, ones having 20 or less carbon atoms are preferably used, ones having 10 to 18 carbon atoms are more preferably used. An alkyl group may be used either a linear chain or a branched chain.

As the hydrophobized polysaccharide, one that primary hydroxyl groups of 1 to 5 per 100 sugars are linked to the polysaccharide following formula (I):-O-(CH₂)ₘCONH(CH₂)ₙNH-CO-O-R (I)(wherein, R represents an alkyl group or a sterol residue; m represents 0 or 1; n represents an arbitrary positive integer) is used preferably. As the alkyl group or the sterol residue, n is preferably used 1 to 8.

As the hydrophobized polysaccharide, one that is linked via a linker can be used.

As the hydrophobized polysaccharide, a non-ionic one is preferably used. The zeta potential of the hydrophobized polysaccharide-based nanogel particles in which the synthetic long peptide antigen or the recombinant protein antigen is loaded is preferably from -2.0 mV to +2.0 mV under physiological conditions. The particle size of the hydrophobized polysaccharide-based nanogel in which the synthetic long peptide antigen or the recombinant protein antigen is loaded is preferably 80 nm or less.

The pretreatment drug of the disclosure that comprises an immune-enhancing agent and a pharmaceutical composition, which contains a hydrophobized polysaccharide-based nanogel as a delivery system in which a synthetic long peptide antigen or a recombinant protein antigen is loaded, may be administered in various ways. Suitable non-oral administered routes, such as intravenous, intraperitoneal, subcutaneous, intradermal, adipose tissue, mammary gland tissue, inhalation or intramuscular, or mucosal route in the form of nasal drops are preferably used.

The pretreatment drug of the disclosure, normally, is prepared as a kit which contains the antigen-loaded nanogel mixed with an immune-enhancing agent or the antigen-loaded nanogel and an immune-enhancing agent separately. The agent may be prepared for a suitable dosage form for subcutaneous, intravenous, and intramuscular administration. The dose of the antigen-loaded nanogel necessary to induce the desired immunity can be appropriately determined. For example, the usual dose can be used in an amount of about 0.1 mg/administration to 10 mg/administration, as the synthetic long peptide antigen or the recombinant protein antigen. The number of times of administration is suitably 2 to 20 times. Dosage intervals between the pretreatment drug and the antigen-specific T cell infusion is selected between 1 day to 2 weeks.

The disclosure provides a pretreatment drug of the therapeutic agent containing the cell population comprising antigen-specific T cells as an active ingredient. The cell population suitable for the treatment of a patient is administered, for example, intravenously injection or infusion, intraarterially, subcutaneously, or intraperitoneally. The cell population can be prepared, according to known methods in the pharmaceutical art, mixed with a known organic or inorganic carrier suitable for non-oral administration, excipients or stabilizers, etc. as drops or injections. The content of the cell population, the dose, other conditions may be appropriately determined according to the known immunotherapy. The content of the cell population in the pharmaceutical, not limited, is preferably 1×10³ to 1×10¹¹ cells/mL, more preferably 1×10⁴ to 1×10¹⁰ cells/mL, more preferably 1×10⁵ to 2×10⁹ cells/mL. The dosage of the therapeutic agent containing the cell population as an active ingredient, not limited, is preferably 1×10⁶ to 1×10¹² cells/day per adult, more preferably 1×10⁷ to 5×10¹¹ cells/day per adult, more preferably 1×10⁸ to 2×10¹¹ cells/day per adult. In the preparing method of the cell population, the step of introducing a foreign gene into the cell population can be included. "A foreign gene" means the gene which is artificially introduced into the cell population containing the target T cell, and also encompasses genes from the same species of the target cells. The means for introducing a foreign gene is not limited, can be selected and used by known gene introduction methods appropriately. Gene transfer can be practiced with a viral vector or without viral vectors. On these methods, many papers have been previously reported.

As virus vectors, not limited, known viral vectors used for gene transfer, for example, such as retroviral vectors, lentiviral vectors, adenoviral vectors, adeno-associated virus vectors, simian viral vectors, vaccinia virus vectors, or Sendai viral vectors or the like can be used. Retroviral vector or lentiviral vector which can incorporate stably a foreign gene into a chromosomal DNA in targeted cells can be preferably used. As virus vectors, those which lacks replication ability can be preferably used so that they can not self-replicate in an infected cell. When a gene is transferred, a reagent for improving the gene transfer efficiency, such as RetroNectin (registered trademark, Takara Bio), can be used. As the gene introduction methods without using viral vectors, methods using carriers such as liposomes or ligand-polylysine, calcium phosphate method, electroporation method, or the particle gun method and the like can be used. In this case, a foreign gene integrated in plasmid DNA, a linear DNA or RNA is introduced.

As the foreign gene that is introduced is not particularly limited, any foreign genes (for example, enzymes, cytokines, chemokines, or antigen receptors such as T-cell receptor (TCR) or chimeric antigen receptor (CAR), genes encoding proteins such as a receptor of co-stimulation or ligand, antisense nucleic acids, siRNA, miRNA, ribozymes, genes encoding aptamers) can be used. Foreign genes, for example, can be used by inserting into a vector or plasmid such as to be expressed under the control of a suitable promoter. Within the vector, regulatory sequences such as enhancer sequence or terminator sequence can be incorporated.

The target of the therapeutic agents using antigen-loaded nanogel, the immune-enhancing agent, and the antigen-specific T cell infusion is a human who has tumors resistant to immune checkpoint inhibitors. Although not limited, the tumors types are exemplified, such as prostate cancer, colon cancer, melanoma, head and neck cancer, esophageal cancer, stomach cancer, colorectal cancer, liver cancer, gallbladder-bile duct cancer, pancreatic cancer, lung cancer, breast cancer, ovarian cancer, bladder cancer, kidney cancer, testicular cancer, bone and soft tissue sarcoma, malignant lymphoma, leukemia, cervical cancer, skin cancer, brain tumors and the like.

Next, the disclosure will be explained in detail with reference to examples. The technical scope of the invention is not limited by these examples and can be carried out in variously forms without changing the summary of the invention.

### <Example 1>

### 1. Materials and Methods

Anti-mouse CD16/CD32 antibody (clone 93), PE-labeled anti-mouse PD-L1 antibody (clone 9G2), APC-Cy7-labeled anti-CD45 antibody (clone 30-F11), and PE-Cy7-labeled anti-PD-1 antibody (clone 29F.1A12) were purchased from Biolegend. V450-labeled anti-CD8 antibody (clone 53-6.7) was purchased from eBioscience. Fetal bovine serum (FBS) was purchased from Bio-West. RPMI1640 medium (containing 2-mercaptoethanol) was purchased from the Cell Science Institute. Erythrocyte hemolysis solution (0.15 M NH₄Cl/10 mM KHCO₃/0.1 mM EDTA.Na₂ pH 7.2) was prepared in Mie University. Mouse colon cancer CT26 cell line (CRL-2638) was purchased from ATCC and was used as subcultured in Mie University. Mouse fibrosarcoma CMS7 cell line and murine fibrosarcoma CMS5a cell line were obtained from Memorial Sloan-Kettering Cancer Institute and were used as subcultured in Mie University. Human NY-ESO-1 antigen gene was obtained from Memorial Sloan-Kettering Cancer Institute. CMS5a-NY cell line which is CMS5a cell line stably transfected with human NY-ESO-1 antigen gene was produced in Mie University, and was used as subcultured. Female BALB/c mice from 6-week-old to 12-week-old were purchased from Japan SLC and housed at Mie University School of Medicine Animal Center. Protocols of animal experiments were approved by the ethics committee of Mie University School of Medicine.

Mouse colon cancer CT26 cell line, BALB/c mice fibrosarcoma CMS7 cell line, mouse fibrosarcoma CMS5a cell line, and CMS5a-NY cell line were cultured in 10% FBS-containing RPMI1640 medium using T75 culture flasks (Corning). Each cell line was detached using 0.5% trypsin-containing phosphate-buffered saline (PBS) from the flasks, and suspended in 10% FBS-containing RPMI1640 medium. The suspension was centrifuged (400×g, 5min, 4°C) to remove the supernatant. The cells were washed twice with RPMI1640 medium and suspended in RPMI1640 medium at a concentration of 1×10⁶/100 pL. The suspension was subcutaneously implanted in the back part of the BALB/c mouse at a dose of 100 pL/individual (3 mice per group).

Each cell line was implanted subcutaneously, tumors were recovered after 1 week. Tumors were stained immunohistochemically in the manner followings. The tumor embedded in the O.C.T. compound (Sakura Finetech) was frozen and sliced with 3 pm thickness. The sliced tumor sections were dried by air for 2 hours. Dried tumor sections were fixed with ice cold acetone for 15 minutes and used for immunostaining. After the tumor sections were washed 3 times with PBS, and immersed in blocking solution (1% bovine serum albumin (BSA) and 5% Blocking One Histo (Nacalai Tesque) containing PBS) at 4°C. Anti-mouse CD16/CD32 antibody was diluted in blocking solution at a concentration of 1 pg/mL. The tumor sections were prepared with the antibody solution for 30 minutes at room temperature in a humidified box to block the Fcy receptor. Next, the tumor sections were stained with PE-labeled anti-mouse PD-L1 antibody diluted at a concentration of 1 pg/mL in blocking solution for 1 hour at room temperature in a humidified box. After the tumor sections were washed three times with 0.02% Tween20-containing PBS, they were immersed in Prolong Gold antifade reagent with DAPI (Life Technologies). The tumor sections were observed with a fluorescent microscope BX53F (Olympus) or confocal laser scanning microscope LSM780 (Carl Zeiss). The microscopic images were processed using Photoshop Element (Adobe Systems).

Each cell line was implanted subcutaneously, and the immune cells infiltrating tumor were separated after 1 week in the following manner. Tumor was isolated from a mouse, crushed by using the Gentle MACS (Miltenyi), and suspended in RPMI1640 medium. At this time, separated cells from 3 mice in a group were pooled. Collagenase D (final concentration 2 mg/mL, Roche) was added to suspended cells, reacted for 30 minutes at 37°C, and the cells were crushed again using the Gentle MACS. The cells were filtrated with a filter (22-pm pore size, BD Biosciences), centrifuged (400×g, 5 min, 4°C), and the supernatant was removed, 2 mL of erythrocyte hemolysis solution was added to the cells. After one minute, 18 mL of RPMI1640 medium were added, and the cells were centrifuged (400×g, 5 min, 4°C). After the supernatant was removed, the cells were suspended in RPMI1640 medium. After counting the number of cells, they were suspended in staining buffer (0.5% BSA-containing PBS) to give a cell concentration of 3×10⁷ cells/mL. Fifty micro liters of the cell suspension per well was transferred in a 96-well V-bottom microplate (Nunc). The microplate was centrifuged (2000 rpm, 1 min, 4°C). After removing the supernatant, the cells were suspended in 50 pL of staining buffer per well. APC-Cy7-labeled anti-mouse CD45 antibody, V450-labeled anti-mouse CD8 antibody, and PE-Cy7-labeled anti-mouse PD-1 antibody were added to cells as recommended use concentration of manufacturer of each antibody. After mixing gently, they were allowed to stand in the dark for 15 minutes at 4°C. The cells were washed twice with 200 pL of staining buffer, suspended in 200 pL of staining buffer, and transferred to a round-bottomed polystyrene tubes (BD Biosciences). The cells were analyzed using a flow cytometer FACS Canto II (BD Biosciences) and data analysis software FlowJo (Tree Star). The frequency of PD-1 expression was determined as expression frequencies (%) in the cell population of CD45-positive and CD8-positive. The frequency of CD8-positive T cells was determined as the frequency (%) of CD8-positive cells in the CD45-positive cell population.

### 2. Results

Immune checkpoint inhibitor-resistant human tumors indicate the features that the immune checkpoint molecules or CD8-positive T cells infiltrating to tumor are not observed (Non-patent Document 5). To find the same characteristics of mouse tumor, after various mouse cancer cell lines were implanted subcutaneously in BALB/c mice, tumors were harvested. Immune checkpoint molecule PD-L1 and PD-1 expression, as well as the infiltration number of CD-positive T cells were measured in the tumors. Figure 1(A) showed the results of the expression of PD-L1 molecules in CT26 tumor, CMS7 tumor, CMS5a-NY tumor and CMS5a tumor analyzed by immunostaining. Many cells expressing PD-L1 were observed in CT26 tumor, the CMS7 tumor and CMS5a-NY tumor, whereas the number of PD-L1-expressing cells in CMS5a tumor was extremely small. Figure 1(B) showed the results of the expression frequency of PD-1 in CD3-positive T cells in tumors by flow cytometry. The percentage of PD-1 expressing CD3-positive T cells in CMS5a tumors was the lowest as compared to other tumors. Figure 1(C) showed the frequency of CD8-positive T cells infiltrating into the tumor localized in each tumor. The frequency of CD8-positive T cells infiltrating the tumor localized in CMS5a tumor was significantly lower as compared to other tumors. From these results, mouse tumor fibrosarcoma formed by implantation by CMS5a cell line subcutaneously in mice was found to exhibit the same characteristics as immune checkpoint inhibitor-resistant human tumors.

### <Example 2>

### 1. Materials and Methods

A hybridoma which expresses anti-mouse CTLA-4 antibody (clone 9D9) was obtained from Dr. James P. Allison in MD Anderson Cancer Center, and the antibody was prepared in Mie University. A hybridoma which expresses anti-mouse GITR antibody (clone DTA-1) was obtained from Dr. Shimon Sakaguchi in Osaka University, and the antibody was prepared in Mie University. Anti-mouse-PD-1 antibody (clone RMP 1-14) was obtained from Dr. Hideo Yagita in Juntendo University. Fetal bovine serum (FBS) was purchased from Bio-West. RPMI1640 medium (containing 2-mercaptoethanol) was purchased from the Cell Science Institute. Mouse colon cancer CT26 cell line (CRL-2638) was purchased from ATCC and was used as subcultured in Mie University. Mouse fibrosarcoma CMS7 cell line and murine fibrosarcoma CMS5a cell line were obtained from Memorial Sloan-Kettering Cancer Institute and were used as subcultured in Mie University. Human NY-ESO-1 antigen gene was obtained from Memorial Sloan-Kettering Cancer Institute. CMS5a-NY cell line which is CMS5a cell line stably transfected with human NY-ESO-1 antigen was produced in Mie University, and was used as subcultured. Female BALB/c mice from 6-week-old to 12-week-old were purchased from Japan SLC and housed at Mie University School of Medicine Animal Center. Protocols of animal experiments were approved by the ethics committee of Mie University School of Medicine.

CT26 cell line, CMS7 cell line, CMS5a cell line, and CMS5a-NY cell line were cultured in 10% FBS-containing RPMI1640 medium using T75 culture flasks (Corning). Each cell line was detached using 0.5% trypsin-containing phosphate buffer saline (PBS) from the flasks, and suspended in 10% FBS-containing RPMI1640 medium. The suspension was centrifuged (400×g, 5 min, 4°C) to remove the supernatant. The cells were washed twice with RPMI1640 medium and suspended in RPMI1640 medium at a concentration of 1×10⁶/100 pL. The suspension was subcutaneously implanted in the back part of the BALB/c mice at a dose of 100 pL/individual (4 mice per group). Anti-mouse PD-1 antibody diluted in PBS (150 pg), anti-mouse CTLA-4 antibody diluted in PBS (100 pg) and anti-mouse GITR antibody diluted in PBS (100 pg) as immune checkpoint inhibitors were intraperitoneally administered simultaneously at 7, 9 and 11 days after tumor implantation. The length and breadth of the tumors were measured after tumor transplantation over time, and the tumor volumes were calculated according to the formula: (longer diameter × shorter diameter × shorter diameter × 0.5). Statistical analysis was performed by non-parametric test using Microsoft Excel (Microsoft).

### 2. Results

From the results in Example 1, tumors formed by subcutaneously implanted murine fibrosarcoma CMS5a cell line in BALB/c mice were expected to be resistant to immune checkpoint inhibitors. Therefore, combination therapy was attempted using anti-PD-1 antibody, anti-CTLA-4 antibody and anti-GITR antibody as immune checkpoint inhibitors to BALB/c mice with tumors formed by subcutaneously implanted mouse cancer cell lines. The results were shown in figure 2. The inhibition effects on tumor growth by the combination therapy using immune checkpoint inhibitors were observed clearly in CT26 tumor, CMS7 tumor and CMS5a-NY tumor. In contrast, the combination therapy using immune checkpoint inhibitors did not have any effect on CMS5a tumor, they showed similar growth as untreated group. Therefore, CMS5a tumor was proved to exhibit strong resistance to immune checkpoint inhibitors. Together with the results of Example 1, CMS5a tumor was considered to be a good model of immune checkpoint inhibitor-resistant human tumors. It became clear that effective treatments for immune checkpoint inhibitor-resistant human tumors are examined by using CMS5a tumor as an evaluation system.

### <Example 3>

### 1. Materials and Methods

Cholesteryl pullulan (abbreviation CHP, trade name CHP-80T) was obtained from NOF Corporation. Incomplete Freund's adjuvant (abbreviation IFA, model number F5506) was purchased from Sigma-Aldrich. Long peptide antigen-loaded CHP nanogel was prepared as follows. Long peptides antigens (MEN peptide: SNPARYEFLYYYYYYQYIHS ANVLYYYYYYRGPESRLL (SEQ ID NO: 1) and p121 peptide: NDHIAYFLY QILRGLQYIHSANVLHRDLKPSNLLLNT (SEQ ID NO: 2)) were chemically synthesized by Bio-Synthesis and were dissolved in dimethyl sulfoxide (abbreviation DMSO, Nacalai Tesque) at a concentration of 10mg/mL. CHP was dissolved in phosphate-buffered saline (PBS) containing 6 M urea (Nacalai Tesque) at a concentration of 10 mg/mL. One mL (10 mg) of the long peptide antigen solution and 20mL (200 mg) of the CHP solution were mixed, and left overnight with gentle stirring at 4°C in the dark. Mixture was transferred to dialysis membrane (molecular weight: 3,500, Thermo Scientific), and dialyzed against PBS containing 0.6 M urea as external dialysis solution in a volume ratio of 100 times or more for 2 hours to overnight at 4°C. Furthermore, dialysis was performed using PBS containing 0.06 M urea as an external dialysis solution in a volume ratio of 100 times for 2 hours to overnight at 4°C. Again, dialysis was performed using PBS as an external dialysis solution in a volume ratio of 100 times or more for 2 hours to overnight at 4°C. The dialysis internal solution was recovered and filtrated by sterilization filter with 0.22pm pore size (PVDF membrane, Millipore). After filtration, the UV absorbance at 280nm was measured using Nanodrop 2000 (Thermo Scientific). The final concentration of the long peptide antigen was determined with the molecular extinction coefficient (1 mg/mL = 4.181).

The long peptide antigen:IFA mixture was prepared as follows. The long peptide antigen was dissolved at a concentration of 60 pg/125 pL in PBS containing 25% DMSO and collected into a syringe. Separately, 125 µL of IFA were taken to another syringe. After both syringes were connected by a three-way stopcock, suctions and discharges by syringes were repeated. After mixing well, the solution was used for administration. Fetal bovine serum (FBS) was purchased from Bio-West. RPMI1640 medium (containing 2-mercaptoethanol) was purchased from the Cell Science Institute. Mouse fibrosarcoma CMS5a cell line was obtained from Memorial Sloan-Kettering Cancer Institute, and was used as subcultured in Mie University. Mouse fibrosarcoma CMS5a cell line expresses mutated ERK2 protein. The peptide containing the mutation site of the mutated ERK2 protein (QYIHSANVL: SEQ ID NO: 3, the underline indicates the mutation) is recognized by the CD8-positive cytotoxic T cells of BALB/c mice. A T cell receptor (TCR) that recognizes the mutant peptide was isolated, and the TCR gene-introduced mouse (DUC18 mouse) has been produced. The long peptide antigens used in the example (MEN peptide and p121 peptide) contain the CD8-positive cytotoxic T-cell recognizing epitope sequence of the mutated ERK2 (QYIHSANVL: SEQ ID NO. 3).

Female BALB/c mice from 6-week-old to 12-week-old were purchased from Japan SLC. DUC18 mice were obtained from the University of Washington, were used as bred at Mie University. The mice were bred at Mie University School of Medicine Animal Center. Protocols of animal experiments were approved by the ethics committee of Mie University School of Medicine.

Mouse fibrosarcoma CMS5a cell line was cultured in 10% FBS-containing RPMI1640 medium using T75 culture flasks (Corning). The cell line was detached using 0.5% trypsin-containing PBS from the flasks and suspended in 10% FBS-containing RPMI1640 medium. The suspension was centrifuged (400×g, 5 min, 4°C) to remove the supernatant. The cells were washed twice with RPMI1640 medium and suspended in RPMI1640 medium at a concentration of 1×10⁶/100 pL. The suspension was subcutaneously implanted in both sides of the back part of BALB/c mouse at a dose of 100 pL/individual (4 mice per group). When the antigen loaded nanogel and an immune enhancer were used as the pretreatment drug, the long peptide antigen-loaded CHP nanogel or the long peptide antigen:IF A mixture with 50 pg of CpG oligo DNA1668 (Gene Design) in PBS as an immune enhancer was administered to the back part subcutaneously or in tail vein of mice at 7 days and 11 days after tumor implantation. In the experiment shown in figure 4(A), p121 peptide was used as a long peptide antigen. In other experiments, MEN peptide was used. CD8-positive T cells in the spleen of mutated ERK2-specific TCR transgenic mice (DUC18 mice) were isolated using CD8a+ T Cell Isolation Kit (Miltenyi). Isolated CD8-positive T cells were suspended to RPMI1640 medium at a concentration of 2×10⁶ cells/200 pL. Isolated CD8-positive T cells were infused from the tail vein as antigen-specific T cells for the treatment after 8 days and 12 days from tumor implantation. Statistical analysis was performed by non-parametric test using Microsoft Excel (Microsoft).

### 2. Results

An effective treatment for immune checkpoint inhibitor-resistant human tumors was searched with the evaluation system using CMS5a tumors formed by implanted subcutaneously in BALB/c mice. As a result, as shown in figure 3(A), the proliferation of CMS5a tumor was significantly inhibited by antigen-specific T cell infusion after subcutaneous administration of the long peptide antigen-loaded CHP nanogel and immune-enhancing agent (CpG oligo DNA) as the pretreatment drug. The therapeutic effect was not observed in the case of using the IFA as the delivery system. As shown in figure 3(B), the intravenous administration of the pretreatment drug, in place of subcutaneous administration, was found to be also effective. As shown in figure 3(C), poly-IC RNA as an immune-enhancing agent in the pretreatment drug, in place of CpG oligo DNA, was found to be effective.

As shown in figure 4(A), the pretreatment drug omitting the long peptide antigen loaded CHP nanogel was found to be not effective. As shown in figure 4(B), the pretreatment drug omitting the immunological enhancer (CpG oligo DNA) was found to be not effective. These results showed that the pretreatment drug of the antigen-specific T cell infusion must contain the long peptide antigen-loaded CHP nanogel and an immune-enhancing agent. As shown in figure 4(C), the pretreatment drug omitting the antigen-specific T cell infusion was found to be not effective.

Thus, the pretreatment drug of the disclosure in combination with antigen-specific T cell infusion was found to treat immune checkpoint inhibitor-resistant tumors.

### <Example 4>

### 1. Materials and Methods

Rhodamine-labeled CHP nanogel was obtained from Dr. Kazunari Akiyoshi in Kyoto University. APC-Cy7-labeled anti-mouse CD45 antibody (clone 30-F11), FITC-labeled anti-mouse CD8 antibody (clone 53-6.7), PE-labeled anti-mouse CD11b antibody (clone M1/70), Pacific blue-labeled anti-mouse F4/80 antibody (clone BM8) and PE-Cy7-labeled anti-mouse CD11c antibody (clone N418) were purchased from Bio Legend. PerCP-Cy5.5-labeled anti-mouse CD4 antibody (clone RM4-5) was purchased from BD Biosciences. APC-labeled anti-mouse B220 antibody (clone RA3-6B2) was purchased from eBioscience. Fetal bovine serum (FBS) was purchased from Bio-West. RPMI1640 medium (containing 2-mercaptoethanol) was purchased from the Cell Science Institute. Erythrocyte hemolysis solution (0.15 M NH₄Cl/10 mM KHCO₃/0.1 mM EDTA.Na₂ pH 7.2) was prepared in Mie University. Mouse fibrosarcoma CMS5a cell line was obtained from Memorial Sloan-Kettering Cancer Institute and was used as subcultured in Mie University. Female BALB/c mice from 6-week-old to 12-week-old were purchased from Japan SLC and housed at Mie University School of Medicine Animal Center. Protocols of animal experiments were approved by the ethics committee of Mie University School of Medicine.

Mouse fibrosarcoma CMS5a cell line was cultured in 10% FBS-containing RPMI1640 medium using T75 culture flasks (Corning). The cell line was detached using 0.5% trypsin-containing phosphate buffer saline (PBS) from the flasks, and suspended in 10% FBS-containing RPMI1640 medium. The suspension was centrifuged (400×g, 5 min, 4°C) to remove the supernatant. The cells were washed twice with RPMI1640 medium. The cells were suspended in RPMI1640 medium at a concentration of 1×10⁶/100 µL, they were implanted subcutaneously into the back part of the BALB/c mice at a dose of 100 pL/individual (4 per group). One mg of Rhodamine-labeled CHP nanogel (10 mg/mL PBS) was administered in the back portion subcutaneously or the tail vein after 7 days from tumor implantation. At the next day after the Rhodamine-labeled CHP nanogel administration, tumor infiltrating immune cells were separated by the following method. Tumor was isolated from a mouse, was crushed with the Gentle MACS (Miltenyi), and was suspended in RPMI1640 medium. Separated cells from 4 mice in a group were pooled. Collagenase D (final concentration 2 mg/ml, Roche) was added to suspended cells, reacted for 30 min at 37°C, and the cells were crushed again using the Gentle MACS. The cells were filtrated with a filter (22-pm pore size, BD Biosciences), centrifuged (400×g, 5 min, 4°C), and the supernatant was removed, 2 mL of erythrocyte hemolysis solution was added to the cells. After one minute, 18 mL of RPMI1640 medium were added, and the cells were centrifuged (400×g, 5 min, 4°C). After the supernatant was removed, the cells were suspended in RPMI1640 medium. The cells were suspended in RPMI1640 medium. After counting the number of cells, they were suspended in staining buffer (0.5% bovine serum albumin-containing PBS) to give a cell concentration of 3×10⁷ cells/mL. Fifty micro liters of the cell suspension per well were transferred in a 96-well V-bottom microplate (Nunc). The microplate was centrifuged (2000 rpm, 1 min, 4°C), after removing the supernatant, the cells were suspended in 50 pL of staining buffer per well. The regional lymph nodes were collected after 18 hours from Rhodamine-labeled CHP nanogel administration. In the case of subcutaneous administration, lymph nodes of the administration site (the inguinal lymph nodes) were collected, in the case of intravenous administration, the tumor draining lymph nodes (inguinal lymph nodes) were collected.

After grinding the lymph nodes using a glass slide, released cells were suspended in RPMI1640 medium. At this time, cells from 4 mice in a group were pooled. The suspension was centrifuged (400×g, 5 min, 4°C) to remove the supernatant, the cells were treated for 1 min by adding 2 mL of erythrocyte hemolysis solution. 18 mL of RPMI1640 medium were added, and the cells were centrifuged (400×g, 5 min, 4°C). After removing the supernatant, the cells were suspended in RPMI1640 medium. The cell suspension was centrifuged (400×g, 5 min, 4°C) and the supernatant was removed. The cells were washed twice with 2% FBS-containing PBS, and suspended. APC-Cy7-labeled anti-mouse CD45 antibody, FITC-labeled anti-mouse CD8 antibody, PerCP-Cy5.5-labeled anti-mouse CD4 antibody, APC-labeled anti-mouse B220 antibody, PE-labeled anti-mouse CD11b antibody, Pacific blue-labeled anti-mouse F4/80 antibody, and PE-Cy7-labeled anti-mouse CD11c antibody were added as recommended use concentration of manufacturer of each antibody to cell suspensions prepared from tumors or lymph nodes. After mixing, they were allowed to stand in the dark for 15 minutes at 4°C. The cells were washed twice with 200 pL of staining buffer, re-suspended in 200 pL of staining buffer, and transferred to a round-bottomed polystyrene tubes (BD Biosciences). The cells were analyzed using a flow cytometer FACS Canto II (BD Biosciences) and data analysis software FlowJo (Tree Star).

T cells were detected as CD45-positive and CD4-positive, or CD45-positive and CD8-positive, B cells were detected as CD45-positive and B220-positive, macrophages were detected as CD45-positive and CD11b-positive and CD11c positive and F4/80-positive. The Rhodamine-positive cells in each immune cells were detected as CHP nanogel uptake cells.

### 2. Results

As shown in figure 3(B), the pretreatment drug of the disclosure showed similar therapeutic effect on the immune checkpoint inhibitor-resistant CMS5a tumors in both subcutaneous and intravenous administration. To elucidate the mechanism of action of the pretreatment drug, the uptake of CHP nanogel into immune cells was measured in lymph nodes and tumor localized, after administered subcutaneously or intravenously of Rhodamine-labeled CHP nanogel to BALB/c mice into which the CMS5a tumors were implanted subcutaneously. As shown in figure 5, subcutaneously administered CHP nanogel was incorporated well into macrophages at the site of administration lymph nodes. On the other hand, intravenously administered CHP nanogel was incorporated well into tumor localized macrophages. Uptake into other immune cells was not observed. It was thought that macrophages in lymph node or tumor incorporated long peptide antigens delivered by CHP nanogel, to present the antigen to the infused antigen-specific T cells and enhance the activity of antigen-specific T cells. When CHP nanogel is administered intravenously, it may be delivered to tumor localized macrophages selectively.

### <Example 5>

### 1. Materials and Methods

Fetal bovine serum (FBS) was purchased from Bio-West. RPMI1640 medium (containing 2-mercaptoethanol) was purchased from the Cell Science Institute. Erythrocyte hemolysis solution (0.15 M NH₄Cl/10 mM KHCO₃/0.1 mM EDTA.Na₂ pH 7.2) was prepared in Mie University. Mouse fibrosarcoma CMS5a cell line was obtained from Memorial Sloan-Kettering Cancer Institute, and was used as subcultured in Mie University. Female BALB/c mice from 6-week-old to 12-week-old were purchased from Japan SLC. Mutated ERK2-specific TCR transgenic mice (DUC18 mice) were obtained from the University of Washington, and were used as bred at Mie University. The mice were bred at Mie University School of Medicine Animal Center. Protocols of animal experiments were approved by the ethics committee of Mie University School of Medicine.

Mouse fibrosarcoma CMS5a cell line was cultured in 10% FBS-containing RPMI1640 medium using T75 culture flasks (Corning). The cell line was detached using 0.5% trypsin-containing PBS from the flasks, and suspended in 10% FBS-containing RPMI1640 medium. The suspension was centrifuged (400×g, 5 min, 4°C) to remove the supernatant. The cells were washed twice with RPMI1640 medium and suspended in RPMI1640 medium at a concentration of 1×10⁶/100 pL. The suspension was subcutaneously implanted in both sides of the back part of BALB/c mouse at a dose of 100 pL/individual (5 mice per group). At 7 days after tumor implantation, the long peptide antigen-loaded CHP nanogel (60 pg as MEN peptide, dissolved in PBS) prepared in the same manner in Example 3, and CpG oligo DNA1668 (50 pg, dissolved into PBS, Gene Design) were mixed and administered into the tail vein. 18 hours later, antigen presenting cells from tumor, lung, liver, spleen and lymph nodes of treated mice were separated by the method shown below.

The isolation kit made by Miltenyi (Tumor Dissociation Kit (Part No. 130-096-730)) for tumor, the isolation kit made by Miltenyi (Lung Dissociation Kit (Part No. 130-095-927)) for lung, and the isolation kit made by Miltenyi (Liver Dissociation kit (Part No. 130-105-807))) for liver, were used respectively. After treatment according to the manufacturer's recommended protocol, isolated cells were suspended in RPMI1640 medium.

At this time, cells from 5 mice in a group were pooled. The suspension was centrifuged (400×g, 5 min, 4°C) to remove supernatant, the cells were treated for 1 min by adding 2 mL of erythrocyte hemolysis solution. 18 mL of RPMI1640 medium were added, and the cells were centrifuged (400×g, 5 min, 4°C). After removing the supernatant, the cells were suspended in RPMI1640 medium. After the spleen and inguinal lymph nodes were triturated with a glass slide, released cells were collected in RPMI1640 medium. At this time, cells from 5 mice in a group were pooled. The suspension was centrifuged (400×g, 5 min, 4°C) to remove the supernatant, the cells were treated for 1 minute by adding 2 mL of erythrocyte hemolysis solution. 18 mL of RPMI1640 medium were added, and the cells were centrifuged (400×g, 5 min, 4°C). After removing the supernatant, the cells were suspended in RPMI1640 medium (it was called "the primary cell suspension"). The primary cell suspension prepared from each tissue was centrifuged (400×g, 5 min, 4°C) and the supernatant was removed. After the cells were washed twice with 2% FBS-containing PBS, they were suspended in 2% FBS-containing PBS. The suspension was called "the secondary cell suspension". CD11b-positive cells were isolated using CD11b microbeads (Miltenyi) from the secondary cell suspension. These cells were used as antigen presenting cells from each tissue. On the other hand, CD8-positive T cells were isolated from the spleen of DUC18 mice in the same manner in Example 3. Then the responder T cells were prepared by labeling with the fluorescent dye CFSE (Thermo Fisher Science). 2.5×10⁵ cells of antigen presenting cells and 2×10⁵ cells of responder T cells per well were added to a 96-well V-bottom microplate (Nunc), and co-cultured for 72 hours in 10% FBS-containing RPMI1640 medium. When the responder T cells proliferate in response to antigen presentation, the fluorescence of CFSE is attenuated with the cell division. The change of the fluorescence was measured using a flow cytometer FACS Canto II (BD Biosciences) and data analysis software FlowJo (Tree Star). The percentage of responder T cells which divided more than once was calculated, and the antigen presenting ability of antigen-presenting cells from each tissue was evaluated.

### 2. Results

In Example 4, it was revealed that intravenously administered CHP nanogel was taken up selectively tumor localized macrophages. It was thought that the long peptide antigen-loaded CHP nanogels administered intravenously were taken into tumor localized macrophages, the antigen was presented to the infused antigen-specific T cells to enhance the activity of antigen-specific T cells. The following experiments were performed to confirm the antigen-presenting activity of tumor localized macrophages. CD11b-positive macrophages in tumor or various tissues were isolated from BALB/c mice in which CMS5a tumor had been implanted subcutaneously, and CHP nanogel loaded with long peptide antigen containing the CD8-positive T cells recognizing epitope of mutated ERK2 and CpG oligoDNA were intravenously administered. The CD11b-positive macrophages as antigen-presenting cells were co-cultured with the CD8-positive T cells from mutated ERK2-specific TCR transgenic mice in vitro. If the CD11b-positive macrophages present the CD8-positive T cell recognizing epitope of the mutated ERK2 derived from the administered long peptide antigen, the CD8-positive T cells from mutated ERK2-specific TCR transgenic mice are activated and proliferated. The fluorescence was measured by CFSE dilution test using flow cytometry to estimate the T cell proliferation and was used as an indicator of antigen presentation.

As shown in figure 6, when a long peptide antigen-loaded CHP nanogel and CpG oligoDNA were administered intravenously, the long peptide antigen was presented by tumor localized macrophages. Macrophages from lymph nodes was observed to present the long peptide antigen weakly. Macrophages from other tissues were not observed to present the long peptides antigen. It was thought that these macrophages did not incorporate the long peptide antigen-loaded CHP nanogel and CpG oligoDNA, or may lack the ability to present the antigen. These results showed that CHP nanogel has the ability to transport molecules, in particular antigen, to tumor localized macrophages selectively and to make the antigen presented, when CHP nanogel was administered intravenously.

The mechanism was considered the same in non-human mammals, containing monkey, mouse, rat, pig, cattle, and dog. The composition of the disclosure was thought to have the same effect on human, monkey, mouse, rat, pig, cattle, dog etc.

According to these examples, it was possible to provide a therapeutic technique for treating the immune checkpoint inhibitor-resistant tumors which do not express the molecular targets of immune checkpoint inhibitors. The enhancement of anti-cancer effect of antigen-specific T cell infusion was derived by a synthetic long peptide antigen or recombinant protein antigen loaded-nanogel using a hydrophobized polysaccharide-based nanogel as the delivery system and immunological enhancer, as a pretreatment drug.

## Claims

1. A pharmaceutical composition for use in a TCR gene-engineered T cell infusion therapy or CAR gene-engineered T cell infusion therapy against an immune checkpoint inhibitor-resistant tumor, administered at least 1 day prior to the administration of the antigen-specific T cells, wherein the pharmaceutical composition comprises:
a long peptide antigen or a protein antigen which contains CD8-positive cytotoxic T cell recognizing epitope(s) and/or CD4-positive helper T cell recognizing epitope(s) derived from the antigen,
a hydrophobized polysaccharide-based nanogel,
an antigen-loaded nanogel which contains the hydrophobized polysaccharide-based nanogel in which the long peptide antigen or the protein antigen is loaded, and
an immuno enhancing agent,
wherein the hydrophobized polysaccharide comprises pullulan and cholesteryl group, and
wherein the immune-enhancing agent is at least one selected from the group consisting of TLR (Toll-like receptor) agonist (CpG oligoDNA or poly-IC RNA), STING agonist, or RLR (RIG-I-like receptors) agonist; and
wherein the antigen-specific T cell is a T cell that expresses a T cell receptor which recognizes the antigen or a chimeric antigen receptor which recognizes the antigen.

2. A pharmaceutical composition comprising antigen-specific T-cells, for use in a TCR gene-engineered T cell infusion therapy or CAR gene-engineered T cell infusion therapy against immune checkpoint inhibitor-resistant tumor, wherein the pharmaceutical composition is administered at least 1 day after the administration of an antigen-loaded nanogel that comprises:
a long peptide antigen or a protein antigen which contains CD8-positive cytotoxic T cell recognizing epitope(s) and/or CD4-positive helper T cell recognizing epitope(s) derived from the antigen,
a hydrophobized polysaccharide-based nanogel, and
an antigen-loaded nanogel which contains the hydrophobized polysaccharide-based nanogel in which the long peptide antigen or the protein antigen is loaded, and
an immuno enhancing agent,
wherein the hydrophobized polysaccharide comprises pullulan and cholesteryl group, and
wherein the immune-enhancing agent is at least one selected from the group consisting of TLR (Toll-like receptor) agonist (CpG oligoDNA or poly-IC RNA), STING agonist, or RLR (RIG-I-like receptors) agonist; and
wherein the antigen-specific T cell is a T cell that expresses a T cell receptor which recognizes the antigen or a chimeric antigen receptor which recognizes the antigen.

3. The pharmaceutical composition for use according to any one of claim 1 or claim 2, wherein the long peptide antigen has 23 to 120 amino acid residues.

4. The pharmaceutical composition according to any one of claims 1 to 3, wherein one selected from the group consisting of 2 to 10 tyrosines, 2 to 10 threonines, 2 to 10 histidines, 2 to 10 glutamines and 2 to 10 asparagines is contained between two epitopes which are contained in the long peptide.

5. The pharmaceutical composition for use according to any one of claims 1 to 4, wherein the antigen is a tumor-specific antigen protein or a tumor stroma-specific antigen protein.

6. The pharmaceutical composition for use according to any one of claims 1 to 5, wherein the route of administration of the antigen-loaded nanogel is at least one selected from the group consisting of subcutaneous, intradermal, intramuscular, intratumoral and intravenous.

## Patentansprüche

1. Pharmazeutische Zusammensetzung zur Verwendung in einer Infusionstherapie einer TCR-Gen-manipulierten T-Zelle oder einer Infusionstherapie mit einer CAR-Gen-manipulierten T-Zelle gegen einen Immuncheckpoint-Inhibitor-resistenten Tumor, die zumindest 1 Tag vor der Verabreichung der antigenspezifischen T-Zellen verabreicht wird, wobei die pharmazeutische Zusammensetzung Folgendes umfasst:
ein langes Peptidantigen oder ein Proteinantigen, das eine CD8-positive zytotoxische T-Zelle, die ein oder mehrere Epitope erkennt, und/oder eine CD4-positive Helfer-T-Zelle, die ein oder mehrere von dem Antigen stammende Epitope erkennt, enthält,
ein Nanogel auf Basis eines hydrophobierten Polysaccharids,
ein antigenbeladenes Nanogel, welches das Nanogel auf Basis eines hydrophobierten Polysaccharids enthält, in welches das lange Peptidantigen oder das Proteinantigen geladen ist, und
ein immunverstärkendes Mittel,
wobei das hydrophobierte Polysaccharid Pullulan und eine Cholesterylgruppe umfasst und
wobei das immunverstärkende Mittel zumindest ein aus der aus einem TLR- (Toll-like-Rezeptor-) Agonisten (CpG-OligoDNA oder Poly-IC-RNA), STING-Agonisten oder RLR-(RIG-I-like-Rezeptor-) Agonisten bestehenden Gruppe ausgewähltes ist; und
wobei die antigenspezifische T-Zelle eine T-Zelle ist, die einen T-Zell-Rezeptor, der das Antigen erkennt, oder einen chimären Antigenrezeptor, der das Antigen erkennt, exprimiert.

2. Pharmazeutische Zusammensetzung, die antigenspezifische T-Zellen umfasst, zur Verwendung in einer Infusionstherapie einer TCR-Gen-manipulierten T-Zelle oder einer Infusionstherapie mit einer CAR-Gen-manipulierten T-Zelle gegen einen Immuncheckpoint-Inhibitor-resistenten Tumor, wobei die pharmazeutische Zusammensetzung zumindest 1 Tag nach der Verabreichung eines antigenbeladenen Nanogels verabreicht wird, Folgendes umfassend:
ein langes Peptidantigen oder ein Proteinantigen, das eine CD8-positive zytotoxische T-Zelle, die ein oder mehrere Epitope erkennt, und/oder eine CD4-positive Helfer-T-Zelle, die ein oder mehrere von dem Antigen stammende Epitope erkennt, enthält,
ein Nanogel auf Basis eines hydrophobierten Polysaccharids und
ein antigenbeladenes Nanogel, welches das Nanogel auf Basis des hydrophobierten Polysaccharids enthält, in welches das lange Peptidantigen oder das Proteinantigen geladen ist, und
ein immunverstärkendes Mittel,
wobei das hydrophobierte Polysaccharid Pullulan und eine Cholesterylgruppe umfasst und
wobei das immunverstärkende Mittel zumindest ein aus der aus einem TLR- (Toll-like-Rezeptor-) Agonisten (CpG-OligoDNA oder Poly-IC-RNA), STING-Agonisten oder RLR-(RIG-I-like-Rezeptor-) Agonisten bestehenden Gruppe ausgewähltes ist; und
wobei die antigenspezifische T-Zelle eine T-Zelle ist, die einen T-Zell-Rezeptor, der das Antigen erkennt, oder einen chimären Antigenrezeptor, der das Antigen erkennt, exprimiert.

3. Pharmazeutische Zusammensetzung zur Verwendung nach einem der Ansprüche 1 oder 2, wobei das lange Peptidantigen 23 bis 120 Aminosäurereste aufweist.

4. Pharmazeutische Zusammensetzung nach einem der Ansprüche 1 bis 3, wobei ein aus der aus 2 bis 10 Tyrosinen, 2 bis 10 Threoninen, 2 bis 10 Histidinen, 2 bis 10 Glutaminen und 2 bis 10 Asparaginen ausgewähltes zwischen zwei Epitopen enthalten ist, die im langen Peptid enthalten sind.

5. Pharmazeutische Zusammensetzung zur Verwendung nach einem der Ansprüche 1 bis 4, wobei das Antigen ein tumorspezifisches Antigenprotein oder ein tumorstromaspezifisches Antigenprotein ist.

6. Pharmazeutische Zusammensetzung zur Verwendung nach einem der Ansprüche 1 bis 5, wobei der Verabreichungsweg des antigenbeladenen Nanogels zumindest ein aus der aus subkutan, intradermal, intramuskulär, intratumoral und intravenös bestehenden Gruppe ausgewählter ist.

## Revendications

1. Composition pharmaceutique à utiliser dans une thérapie par perfusion de lymphocytes T modifiés par gène TCR ou une thérapie par perfusion de lymphocytes T modifiés par gène CAR à l'encontre d'une tumeur résistante aux inhibiteurs de point de contrôle immunitaire, administrée au moins 1 jour avant l'administration des lymphocytes T spécifiques à un antigène, dans laquelle la composition pharmaceutique comprend :
un antigène peptidique long ou un antigène protéique qui contient des lymphocytes T cytotoxiques CD8-positifs reconnaissant un ou des épitopes et/ou des lymphocytes T auxiliaires CD4-positifs reconnaissant un ou des épitopes dérivés de l'antigène,
un nanogel à base de polysaccharide hydrophobisé,
un nanogel chargé d'antigène qui contient le nanogel à base de polysaccharide hydrophobisé dans lequel l'antigène peptidique long ou l'antigène protéique est chargé, et
un agent de renforcement de l'immunité,
dans laquelle le polysaccharide hydrophobisé comprend du pullulane et un groupe cholestéryle, et
dans laquelle l'agent de renforcement de l'immunité est au moins un agent choisi dans le groupe constitué par un agoniste de TLR (Toll-like receptor) (CpG oligoADN ou poly-IC ARN), un agoniste de STING ou un agoniste de RLR (récepteurs RIG-I-like) ; et
dans laquelle le lymphocyte T spécifique à un antigène est un lymphocyte T qui exprime un récepteur de lymphocyte T qui reconnaît l'antigène ou un récepteur d'antigène chimérique qui reconnaît l'antigène.

2. Composition pharmaceutique comprenant des lymphocytes T spécifiques à un antigène, à utiliser dans une thérapie de perfusion de lymphocytes T modifiés par gène TCR ou une thérapie de perfusion de lymphocytes T modifiés par gène CAR à l'encontre d'une tumeur résistante aux inhibiteurs de point de contrôle immunitaire, dans laquelle la composition pharmaceutique est administrée au moins 1 jour après l'administration d'un nanogel chargé d'antigène qui comprend :
un antigène peptidique long ou un antigène protéique qui contient des lymphocytes T cytotoxiques CD8-positifs reconnaissant un ou des épitopes et/ou des lymphocytes T auxiliaires CD4-positifs reconnaissant un ou des épitopes dérivés de l'antigène,
un nanogel à base de polysaccharide hydrophobisé, et
un nanogel chargé d'antigènes qui contient le nanogel à base de polysaccharide hydrophobisé dans lequel est chargé l'antigène peptidique long ou l'antigène protéique, et
un agent de renforcement de l'immunité,
dans laquelle le polysaccharide hydrophobisé comprend du pullulane et un groupe cholestéryle, et
dans laquelle l'agent de renforcement de l'immunité est au moins un agent choisi dans le groupe constitué par un agoniste de TLR (Toll-like receptor) (CpG oligoADN ou poly-IC ARN), un agoniste de STING ou un agoniste de RLR (récepteurs RIG-I-like) ; et
dans laquelle le lymphocyte T spécifique à un antigène est un lymphocyte T qui exprime un récepteur de lymphocyte T qui reconnaît l'antigène ou un récepteur d'antigène chimérique qui reconnaît l'antigène.

3. Composition pharmaceutique à utiliser selon l'une quelconque des revendications 1 ou 2, dans laquelle l'antigène peptidique long présente 23 à 120 résidus d'acides aminés.

4. Composition pharmaceutique selon l'une quelconque des revendications 1 à 3, dans laquelle une combinaison choisie dans le groupe constitué de 2 à 10 tyrosines, de 2 à 10 thréonines, de 2 à 10 histidines, de 2 à 10 glutamines et de 2 à 10 asparagines est contenue entre deux épitopes qui sont contenus dans le peptide long.

5. Composition pharmaceutique à utiliser selon l'une quelconque des revendications 1 à 4, dans laquelle l'antigène est une protéine antigénique spécifique à une tumeur ou une protéine antigénique spécifique au stroma tumoral.

6. Composition pharmaceutique à utiliser selon l'une quelconque des revendications 1 à 5, dans laquelle la voie d'administration du nanogel chargé d'antigène est au moins une choisie dans le groupe comprenant sous-cutanée, intradermique, intramusculaire, intratumorale et intraveineuse.
